(19) Europäisches Patentamt
European Patent Office
Office européen des brevets

(11) Publication number: **0 155 613**
**A2**

(12) # EUROPEAN PATENT APPLICATION

(21) Application number: 85102721.9

(22) Date of filing: 09.03.85

(51) Int. Cl.[4]: C 07 C 131/00
C 07 D 231/12, C 07 D 307/42
C 07 D 307/12, A 01 N 37/36
A 01 N 43/56, A 01 N 43/08

(30) Priority: 22.03.84 US 592056

(43) Date of publication of application: 25.09.85 Bulletin 85/39

(84) Designated Contracting States: AT BE CH DE FR IT LI LU NL SE

(71) Applicant: PPG INDUSTRIES, INC.
One PPG Place
Pittsburgh Pennsylvania 15272(US)

(72) Inventor: Krass, Dennis Keith
507 Dan Avenue
Canal Fulton Ohio 44614(US)

(72) Inventor: Lin, Horng-Jau
413 Tulip Trail
Wadsworth Ohio 44281(US)

(74) Representative: Sternagel, Hans-Günther, Dr. et al,
Patentanwälte Dr. M. Hann Dr. H.-G. Sternagel
Marburger Strasse 38
D-6300 Giessen(DE)

(54) **Diphenylether oxime ester derivatives.**

(57) This invention relates to certain diphenylether oxime ester derivatives, formulations containing said derivatives and the use thereof for preemergence or postemergence control of noxious plants, i.e., weeds.

EP 0 155 613 A2

DIPHENYLETHER OXIME ESTER DERIVATIVES

## Field of the Invention

This invention relates to certain diphenylether oxime ester derivatives, formulations of said derivatives and the use thereof for preemergence or postemergence control of noxious plants, i.e., weeds.

## Description of the Invention

This invention provides herbicidally active diphenylether oxime ester derivatives represented by the Formula I:

I.

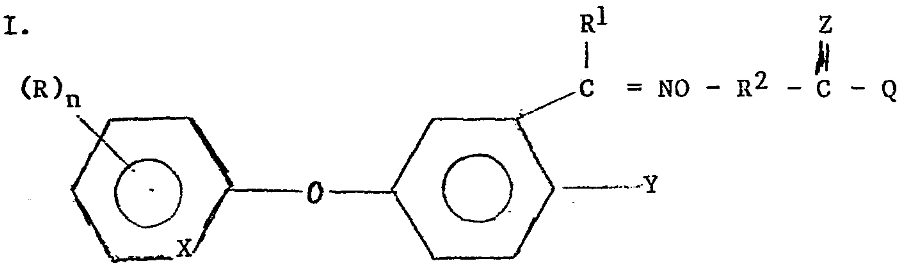

wherein:

X is -CH - or -N-;

Y is nitro, halo or cyano;

Z is oxygen or sulfur;

R is halo, nitro, cyano, alkyl, haloalkyl, alkylthio, haloalkylthio, alkoxy, haloalkoxy, sulfonamide, dialkylsulfonamide, alkyl sulfonyl, haloalkyl sulfonyl, alkylsulfinyl or haloalkylsulfinyl; and n is 1, 2 or 3;

$R^1$ is hydrogen, halo, cyano, alkyl, haloalkyl, cyanoalkyl, alkoxy, haloalkoxy, cyanoalkoxy, alkylthio, haloalkylthio, cyanoalkylthio, mono or dialkylamino, alkylthioalkyl, mono or dialkylaminoalkyl;

$R^2$ is $C_1$ to $C_6$ alkylene or alkenyl that may be substituted by alkyl, haloalkyl, cyanoalkyl or hydroxy; and

Q is $-OR^3$ or $-SR^3$ wherein $R^3$ is alkoxyalkyl, thioalkyl, cyanoalkyl, cycloalkyl, hydroxyalkyl, carboalkoxyalkyl, alkylthioalkyl, aralkyl, sulfonamide, or a 4 to 6 membered heterocyclic ring containing up to 3 hetero atoms or alkyl substituted by a heterocyclic ring containing up to 3 hetero atoms.

Preferred compounds of the invention within the scope of Formula I are those compounds wherein:

R is halogen or haloalkyl and n is 2 or 3;

$R^1$ is alkyl or haloakyl;

$R^2$ is $-\overset{R^4}{\underset{R^5}{C}}-$ wherein $R^4$ or $R^5$ are hydrogen or alkyl;

X is - CH -;

Y is nitro or halo;

Z is oxygen; and

Q is $-OR^3$.

Particularly preferred compounds of the invention within the scope of Formula I are those compounds represented by the Formula II:

II. 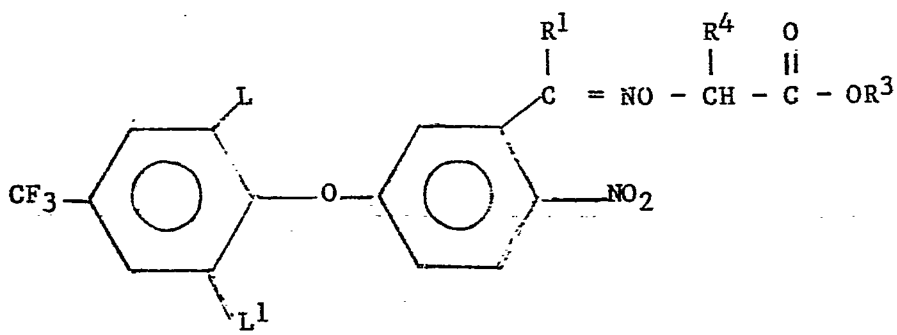

wherein $R^1$, $R^3$ and $R^4$ are as defined with reference to the preferred compounds and L and $L^1$ are the same or different and represent hydrogen or halogen, provided that at least one of L or $L^1$ is halogen.

Alkyl or the alkyl moiety of the substituted alkyl radicals represented by the various "R" groups in the above formulae may be linear or branched and may contain up to 10 carbon atoms, preferably up to 4 carbon atoms. Halo substituents are selected from chlorine, fluorine, bromine or iodine, particularly chlorine, fluorine or bromine. Oxygen, nitrogen and sulfur are exemplary of suitable hetero atoms.

The compounds of this invention may be prepared using techniques and starting materials known and available to the art. For example, compounds of the invention may be prepared by reacting a suitably substituted acid or thioacid halide of the formula:

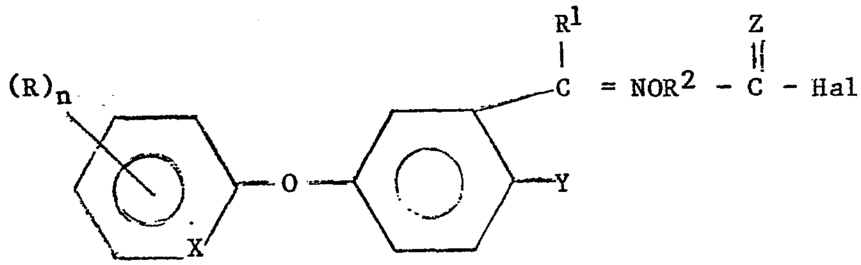

with a suitably substituted alcohol or thiol of the formula, $HOR^3$ or $HSR^3$, wherein R, or, $R^1$, $R^2$, X, Y and Z are as previously defined, and Hal is halogen, e.g., bromine or chlorine. Alternatively, the compounds of the invention may be prepared by a transesterification reaction wherein the said alcohol or thiol is reacted with a suitably substituted ester or thio ester of the formula:

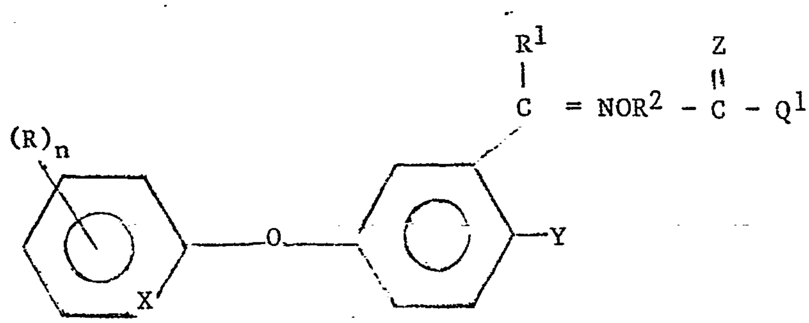

wherein $Q^1$ is $-OR^6$ or $-SR^6$ wherein $R^6$ is preferably lower alkyl, i. e. alkyl of up to 4 carbon atoms, the remaining substituents being as previously defined.

Preparation of certain compounds of the invention by the above generally described modes of preparation are illustrated by the following Examples:

## Example I

Preparation of: 5-(2-chloro-4-trifluoromethyl phenoxy)-2-nitro-acetophenone oxime-O-(acetic acid, 1-(carboethoxy)ethyl ester).

A flask was charged with 0.65 gram (0.0055 mole) of ethyl lactate, 10 milliliters of dry carbon tetrachloride and 0.56 gram (0.0055 mole) of triethylamine. To this stirred solution, at ambient temperature, was slowly added over a period of about 25 minutes, an equivalent amount of 5-(2-chloro-4-trifluoromethylphenoxy)-2-nitroacetophenone oxime-O-acetyl chloride. A granular precipitate formed almost immediately after addition was started. The reaction mixture was stirred overnight at ambient temperature after which the reaction flask was suspended in a 40°C. oil bath and stirring was continued for about 2 days, the progress of the reaction being monitored by TLC. The reaction mixture was then poured into water and phase separated, the aqueous layer being extracted with methylene chloride. The combined organic layers were washed with water and dried over anhydrous magnesium sulfate. The reaction mixture was then filtered and stripped of solvent affording 2.73 grams of brown oil. The oil was dissolved in about 4 milliliters of a 45:55 V/V mixture of ethyl lactate: hexane and added to the top of a column containing 90 grams of silica gel wet-packed with the ethyl lactate:hexane solvent mixture. The column

was eluted the ethyl lacetate:hexane solvent mixture and fractions were automatically collected. Appropriate fractions, after being combined and stripped of solvent, afforded 1.11 grams of brown oil identfied by NMR analysis as the desired product.

## Example II

Preparation of: 5-(2-chloro-4-trifluoromethylphenoxy)-2-nitroacetophenone oxime-O-(acetic acid, 1-methyl-pyrazolyl ester).

A solution of 0.5 gram (0.005 mole) of triethylamine and 0.44 gram (0.0045 mole) of 1-hydroxymethylpyrazole in 20 milliliters of 1:1 V/V methylene chloride:carbon tetrachloride was treated with 0.005 mole of 5-(2-chloro-4-trifluoro methyl phenoxy)-2-nitroacetophenone oxime-O-acetyl chloride over a 17 minute period. An exotherm to 29°C. was observed, along with formulation of a precipitate. After stirring overnight at ambient temperature, TLC analysis indicated some unreacted acid chloride so an additional 0.06 gram of 1-hydroxymethyl pyrazole was added and stirring at ambient temperature was continued for another day. The reaction mixture was then diluted with water and phase separated. The organic phase as washed with water, saturated sodium chloride solution and dried over anhydrous magnesium sulfate. Filtration and solvent stripping afforded 2.93 grams of an organic oil. 2.0 grams of this oil were dissolved in 8 milliliters of a 1:2 V/V mixture of ethyl lactate:hexane and added to the top of column containing 95 grams of silica gel wet-packed with the ethyl lacetate:hexane solvent mixture. The column was eluted with the ethyl lacetate:hexane solvent mixture and 5 milliliters fractions were automatically collected. Appropriate fractions, after being combined and stripped of solvent, afforded 0.74 gram of orange oil identified by NMR and MS analyses as the desired product.

## Example III

Preparation of: 5-(2-chloro-4-trifluoromethylphenoxy)-2-nitroacetophenone oxime-O-(acetic acid, furfuryl ester).

A 25 milliliter, round bottom, 3-necked flask was charged, under anhydrous conditions, with 5.68 grams (0.058 mole) of furfuryl alcohol and 0.01 gram of metallic sodium. To the resulting amber solution was added 1.0 gram (0.0022 mole) of 5-(2-chloro-4-trifluoromethylphenoxy)-2-nitroacetophenone oxime-O-(acetic acid, methyl ester). After stirring for 15 hours at ambient temperature, the reaction mixture was poured into ice water and allowed to phase separate. The organic phase was taken with 20 milliliters of methylene chloride washed 3 x 20 milliliter portions of water and then with saturated sodium chloride solution. After drying over anhydrous sodium sulfate, the organic phase was filtered and stripped of solvent affording 0.8 gram of an orange oil identified by NMR and IR analyses as the desired product.

## Example IV

Preparation of: 5-(2-chloro-4-trifluoromethylphenoxy)-2-nitroacetophenone oxime-O-(acetic acid, tetrahydrofurfuryl ester).

To a 50 milliliter, round-bottom flask has charged 5.27 grams (0.0516 mole) of tetrahydrofurfuryl alcohol and 0.05 gram of p-toluene sulfonic acid. To this stirred mixture, at ambient temperature, was added 1.27 grams (0.00284 mole) of 5-(2-chloro-4-trifluoromethylphenoxy)-2-nitroacetophenone oxime-O-(acetic acid, methyl ester). The reaction mixture was stirred under a vacuum of about 100 millimeters Hg for 2 hours at 75-80°C. and 15 hours at 90°C. The reaction mixture was then stripped of solvent affording a viscous yellow oil which was dissolved in 10 milliliters

of chloroform. After consecutive washings with water (2 x 10 milliliters) 5 percent sodium bicarbonate solution (1 x 10 milliliters), water (1 x 10 milliliters) and saturated sodium chloride solution, the organic phase as dried over anhydrous sodium sulfate, filtered and stripped of solvent affording 1.58 grams of a light yellow, viscous oil identified by NMR analyses as the desired product.

Example V

Preparation of: 5-(2-chloro-4-trifluoromethylphenoxy)-2-nitroacetophenone oxime-O-(acetic acid, ortho-methylbenzl ester).

A reaction mixture consisting of 1.5 grams (0.00336 mole) of 5-(2-chloro-4-trifluoromethylphenoxy)-2-nitroacetophenone oxime-O-(acetic acid, methyl ester), 5.0 grams (0.04093 mole) of ortho-methylbenzyl alcohol and 0.05 gram of p-toluenesulfonic acid was heated, with stirring, at 90-100°C. under a vacuum of about 100 millimeters Hg. The reaction mixture was then cooled and evaporated at 80°C. under a high vacuum affording 4.88 grams of solid material. The solid was dissolved in hot water and the organic layer decanted affording 2.05 grams of crude product. The crude product was dissolved in methylene chloride and added to the top of a column containing 100 grams of silica gel wet-packed with methylene chloride. The column was eluted with 500 milliliters of methylene chloride, 15 milliliter fractions being collected. Appropriate fractions, after being combined and stripped of solvent, afforded 0.90 grams of material identified by NMR and IR analyses as the desired product.

Example VI

Preparation of: 5-(2-chloro-4-trifluoromethylphenoxy)-2-nitroacetophenone oxime-O-(acetic acid, ortho-chlorobenzyl ester).

A reaction mixture consisting of 1.5 grams (0.00336 mole) of 5-(2-chloro 4-trifluoromethylphenoxy)-2-nitroacetophenone oxime -O-(acetic acid, methyl ester), 2.5 grams (0.0125 mole) of ortho-chlorobenzyl alcohol and 0.05 gram of p-toluensulfonic acid was heated, with stirring at 90 - 100°C. under a vacuum of about 100 millimeters Hg. The reaction was then cooled and evaporated at 80°C. under high vacuum affording 2.5 grams of dark brown viscous oil. A 0.2 gram fraction of the crude product was purified by preparative thin layer chromatography using silica gel and 1:20 V/V mixture of ethyl alcohol:methylene chloride affording 0.16 gram of pure material identified by NMR and IR analyses as the desired product. The remaining fraction of crude product was dissolved in methylene chloride and added to the top of a column containing 100 grams of silica gel wet-packed with methylene chloride. The column was eluted with 500 milliliters of methylene chloride, 15 milliliter fractions being collected. Appropriate fractions, after being combined and stripped of solvent, afforded 1.51 grams of colorless, viscous oil identified by NMR and IR analyses as the desired product.

Although preparation of certain compounds of the invention have been illustrated in some detail by the foregoing, it is to be understood that other compounds of the invention may be readily prepared by those skilled in the art using the same or similar techniques and by varying the choice of starting materials.

Weed control in accordance with this invention is effected by application, either before or after emergence of weeds, of a herbicidally

effective amount of a compound of this invention. It is, of course, to be understood that the term "a compound of this invention" also includes mixtures of such compounds.

The term "herbicidally effective amount" is that amount of a compound of this invention required to so injure or damage weeds such that the weeds are incapable of recovering following application. The quantity of a compound of this invention applied in order to exhibit a satisfactory herbicidal effect may vary over a wide range and depends on a variety of factors, such as, for example, hardiness of a particular weed species, extent of weed infestation, climatic conditions, soil conditions, method of application, and the like. Typically, as little as one or less pound per acre of a compound of this invention would be expected to provide satisfactory weed control, although in some instances application rates in excess of one pound per acre; e.g., up to 5 or more pounds per acre might be required. Of course, the efficacy of a particular compound against a particular weed species may readily be determined by routine laboratory or field testing in a manner well known to the art. It is expected that satisfactory weed control can be had at a rate of application in the range of 0.1 to 1.0 pound per acre.

Of course, a compound of this invention can be formulated according to routine methods with any of several known and commonly used herbicidal diluents, adjuvants and carriers. The formulations can contain liquid carriers and adjuvants such as organic solvents, as well as emulsifiers, stabilizers, dispersants, suspending agents, spreaders, penetrants, wetting agents and the like. Typical carriers utilized in dry formulations include clay, talc, diatomaceous earth, silica and the like. Preferred formulations are those in the form of wettable powders, flowables, dispersible

granulates or aqueous emulsifiable concentrates which can be diluted with water at the site of application. Also, dry formulations such as granules, dusts, and the like, may be used.

When desired, a compound of this invention can be applied in combination with other herbicidal agents in an effort to achieve even broader vegetative control. Typical herbicides which can be conveniently combined with Formula I compound include atrazine, hexazinone, metribuzin, ametryn, cyanazine, cyprazine, prometon, prometryn, propazine, simazine, terbutryn, propham, alachlor, acifluorfen, bentazon, metolachlor and N,N-dialkyl thiocarbamates such as EPTC, butylate or vernolate. These, as well as other herbicides described, for example, in the Herbicide Handbook of the Weed Science Society of America, may be used in combination with a compound or compounds of the invention. Typically such formulations will contain from about 5 to about 95 percent by weight of a compound of this invention.

The herbicidal formulations contemplated herein can be applied by any of several methods known to the art. Generally, the formulation will be surface applied as an aqueous spray. Such application can be carried out by conventional ground equipment, of if desired, the sprays can be aerially applied. Soil incorporation of such surface applied herbicides is accomplished by natural leaching, and is of course facilitated by natural rainfall and melting snow. If desired, however, the herbicides can be incorporated into the soil by conventional tillage means.

The compounds prepared as described in the Examples were individually screened for herbicidal efficacy, against a variety of broadleaf and grassy weed species, under controlled laboratory conditions of light, humidity and temperature. Solvent solutions of said compounds were applied, both preemergence and postemergence, to test flats containing the various

weed species, and herbicidal efficacy was determined by periodic visual inspection, after application of the compounds. Herbicidal efficacy was determined on a scale of from 0 (no injury) to 10 (all plants dead).

Basis, these screening tests, of this invention are believed effective for preemergence or postemergence control of a wide variety of broadleaf and grassy weeds. Typical of the various species of vegetative growth that may be controlled, combated, or eliminated are, for example, annuals such as pigweed, lambsquarters, foxtail, crabgrass, wild mustard, field pennycress, ryegrass, goose grass, chickweed, wild oats, velvetleaf, purslane, barnyardgrass, smartweed, knotweed, cocklebur, kochia, medic, ragweed, hemp nettle, spurrey, pondweed, carpetweed, morningglory, duck-salad, cheatgrass, fall panicum, jimsonweed, witchgrass, watergrass, wild turnip, and similar annual grasses and weeds. Biennials that may be controlled include wild barley, campion, burdock, bull thistle, roundleaved mallow, purple star thistle, and the like. Also controlled by the compounds of this invention are perennials such as quackgrass, Johnsongrass, Canada thistle, curly dock, field chickweed, dandelion, Russian knapweed aster, horsetail ironweed, sesbania, cattail, wintercress, horsenettle, nutsedge, milkweed, sicklepod, and the like.

Although the invention has been described in considerable detail by the foregoing, it is to be understood that many variations may be made therein by those skilled in the art without departing from the spirit and scope of thereof, as defined by the appended claims.

CLAIMS:

1. A compound of the formula:

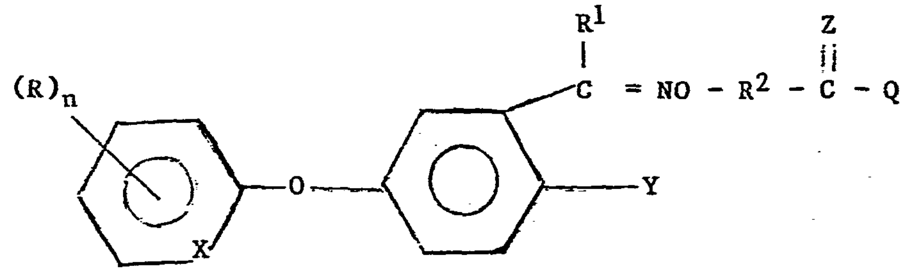

wherein:

X is -CH - or -N-;

Y is nitro, halo or cyano;

Z is oxygen or sulfur;

R is halo, nitro, cyano, alkyl, haloalkyl, alkylthio, haloalkylthio, alkoxy, haloalkoxy, sulfonamide, dialkylsulfonamide, alkyl sulfonyl, haloalkyl sulfonyl, alkylsulfinyl or haloalkylsulfinyl; and n is 1, 2 or 3;

$R^1$ is hydrogen, halo, cyano, alkyl, haloalkyl, cyanoalkyl, alkoxy, haloalkoxy, cyanoalkoxy, alkylthio, haloalkylthio, cyanoalkylthio, mono or dialkylamino, alkylthioalkyl, mono or dialkylaminoalkyl;

$R^2$ is $C_1$ to $C_6$ alkylene or alkenyl that may be substituted by alkyl, haloalkyl, cyanoalkyl or hydroxy; and

Q is $-OR^3$ or $-SR^3$ wherein $R^3$ is alkoxyalkyl, thioalkyl, cyanoalkyl, cycloalkyl, hydroxyalkyl, carboalkoxyalkyl, alkylthioalkyl, aralkyl, sulfonamide, or a 4 to 6 membered heterocyclic ring containing up to 3 hetero atoms or alkyl substituted by a heterocyclic ring containing up to 3 hetero atoms.

2. A compound of the claim 1 wherein:

R is halogen or haloalkyl and n is 2 or 3;

$R^1$ is alkyl or haloalkyl;

$R^2$ is 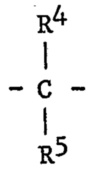 wherein $R^5$ and $R^4$ are hydrogen or alkyl;

X is - CH -;

Y is nitro or halo;

Z is oxygen; and

Q is - $OR^3$.

3. A compound of claim 2 of the formula:

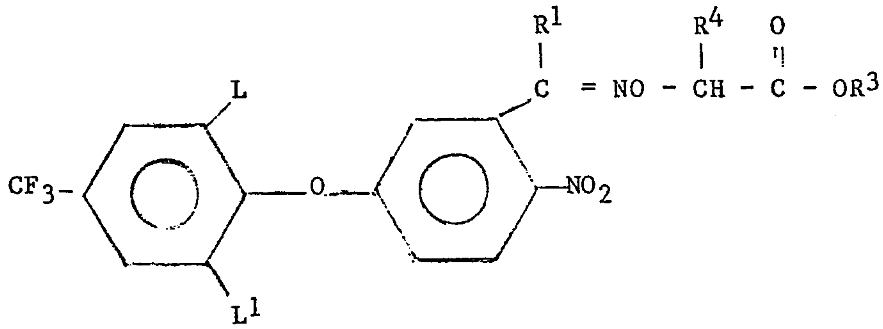

wherein L and $L^1$ are hydrogen or halogen provided that at least one of L or $L^1$ is halogen.

4. A herbicidal composition containing an inert carrier and a herbicidally effective amount of a compound or mixture of compounds defined in claim 1.

5. The method of controlling the growth of weeds wherein a herbicidally effective amount of herbicide is applied to a growth medium prior to emergence of weeds therefrom or to the weeds subsequent to emergence from the growth medium wherein the improvement resides in using as the herbicide a compound or mixture of compounds as defined in claim 1.